# EUROPÄISCHE PATENTSCHRIFT

(11) **EP 0 467 186 B1**
(45) Veröffentlichungstag und Bekanntmachung des Hinweises auf die Patenterteilung: **27.04.1994**
(21) Anmeldenummer: 91111321.5
(22) Anmeldetag: 08.07.1991
(51) Int. Cl.: A61F 13/00, A61F 5/01

(54) **Schultergelenkbandage**
Bandage for shoulder articulation
Bandage pour l'articulation de l'épaule

(30) Priorität: 20.07.1990 DE 9010801 U
(43) Veröffentlichungstag der Anmeldung: 22.01.1992
(73) Patentinhaber: Bauerfeind GmbH & Co., D-47880 Kempen (DE)
(72) Erfinder: Bauerfeind, Hans B., W-4152 Kempen 1 (DE)
(74) Vertreter: Altenburg, Udo, Dipl.-Phys.

(56) Entgegenhaltungen:
- EP-A- 0 143 132
- EP-A- 0 275 459
- DE-C- 328 915
- DE-U- 8 913 490

## Beschreibung

Die Erfindung betrifft eine Schultergelenkbandage mit einem den Oberarm umgreifenden schlauchförmigen Abschnitt aus einem elastischen Material und einem sich an den schlauchförmigen Abschnitt anschließenden, die Schulter abdeckenden kappenförmigen Abschnitt aus einem elastischen Material, ferner mit einem im äußeren Arm bereich des dem kappenförmigen Abschnitt entfernten Randes des schlauchförmigen Abschnittes an diesen angesetzten, nach vorne wendelförmig um den schlauchförmigen Abschnitt herum unter der Achselhöhle der zu bandasierenden Schulter hindurch und an dem hinteren Seitenrand des kappenförmigen Abschnitts entlang u. sodann über die zu bandasierende Schulter hinwes nach vorne über die Brust verlaufenden ersten Extensionsgurt und mit einem im äußeren Arm bereich des dem kappenförmigen Abschnitt entfernten Randes des schlauchförmigen Abschnitts an diesen angesetzten, nach hinten gegenläufig zu dem ersten Extensionsgurt wendelförmig um den schlauchförmigen Abschnitt und am vorderen Seitenrand des kappenförmigen Abschnitts entlang und sodann über die zu bandasierende Schulter hinwes unter Kreuzen des ersten Extensionsgurtes über den Rücken verlaufenden und mit dem ersten Extensionsgurt verbindbaren zweiten Extensionsgurt, wobei die Extensionsgurte im Bereich ihrer Kreuzung bzw. ihrer Verbindung miteinander vernäht sind und jeder Extensionsgurt einen an die Verbindung anschließenden Verbindungsabschnitt aufweist, wobei ein Verbindungsabschnitt den Rücken und der andere Verbindungsabschnitt die Brust überspannt.

Eine derartige Schultergelenkbandage ist bekannt (EP 02 75 459). Sie dient zur Nachbehandlung von operativen Eingriffen am Schultergelenk oder Schultereckgelenk, bei Rotatorenmanschettenrupturen, schmerzhafter Schultersteife, bei subcapitaler Humerusfraktur, Schulterluxation oder bei einer Schultereckgelenksprengung, um Schmerzen zu lindern und den Heilungsprozeß zu unterstützen. Die bekannte Schultergelenkbandage hat sich an sich bewährt. Allerdings hat sich gezeigt, daß der Tragekomfort dadurch beeinträchtigt werden kann, daß bei der bekannten Schultergelenkbandage die Extensionsgurte direkt unter bzw. in der Achselhöhle der anderen Schulter verlaufen.

Aufgabe der Erfindung ist es, den Tragekomfort einer gattungsgemäßen Schultergelenkbandage zu verbessern.

Dadurch wird vermieden, daß die Extensionsgurte oder ihre Kreuzung unmittelbar im Bereich der Achselhöhle angeordnet sind, die den Rücken bzw. die Brust überspannenden Verbindungsabschnitte ziehen die Extensionsgurte und ihre Kreuzung soweit aus dem Bereich der Achselhöhle, daß diese nicht mehr beansprucht wird.

In an sich bekannter Weise kann vorgesehen sein, daß wenigstens das Material des schlauchförmigen Abschnitts längselastisch ausgebildet ist. Die beiden Verbindungsabschnitte können über ein eine Längsverstellung ermöglichendes Verschlußstück miteinander verbunden sein. Ferner kann der schlauchförmige Abschnitt und/oder der kappenförmige Abschnitt mit die Extensionsgurte aufnehmende Taschen versehen sein. Es besteht auch die Möglichkeit, daß der kappenförmige Abschnitt im Bereich des Schultergelenks mit einer zur Aufnahme einer Pelotte dienende Tasche versehen ist.

Im folgenden wird ein in der Zeichnung dargestelltes Ausführungsbeispiel der Erfindung erläutert; es zeigen:

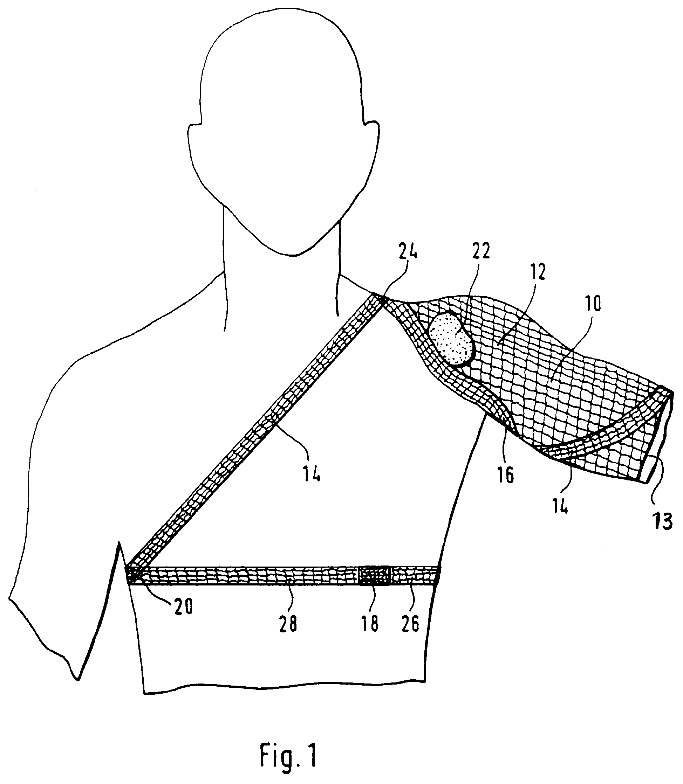

- Fig. 1: die Schultergelenkbandage in einer Ansicht von vorne,

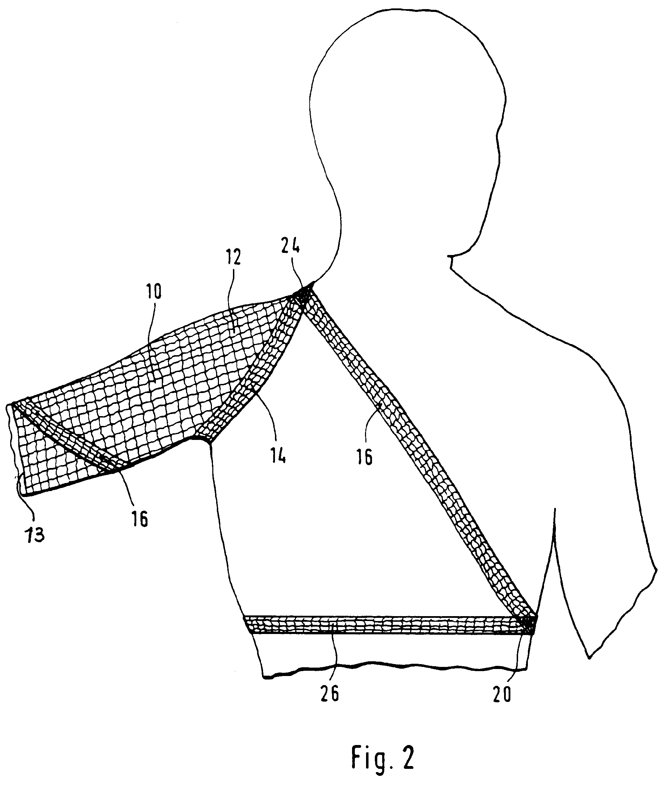

- Fig. 2: die Schultergelenkbandage in einer Ansicht von hinten.

Die Schultergelenkbandage besteht aus einem den Oberarm umgreifenden schlauchförmigen Abschnitt 10 aus einem elastischen Material, einem an diesen angesetzten vorzugsweise einstückig gestrickten, die Schulter abdeckenden kappenförmigen Abschnitt 12, der ebenfalls aus einem elastischen Material besteht sowie zwei Extensionsgurte 14, 16.

Der erste Extensionsgurt 14 ist im oberen Bereich des dem kappenförmigen Abschnitt 12 entfernten Randes 13 des schlauchförmigen Abschnitts 10, oberhalb des Ellenbogens, an den schlauchförmigen Abschnitt 10 angesetzt und verläuft zunächst nach vorne wendelförmig um den schlauchförmigen Abschnitt 10 herum, unter der Achselhöhle der zu bandagierenden Schulter hindurch, an dem hinteren Seitenrand des kappenförmigen Abschnitts 12 entlang sowie dann über die zu bandagierende Schulter und schräg über die Brust zu einer Kreuzung (20), die sich unter der anderen Schulter befindet.

Der zweite Extensionsgurt 16 verläuft von dem kappenförmigen Abschnitt 12 abgewandten Rand 13 des schlauchförmigen Abschnitts 10 im äußeren Armbereich zunächst nach hinten wendelförmig um diesen herum, sodann entlang des vorderen Randes des kappenförmigen Abschnittes über die Zu bandagierende Schulter hinweg, den ersten Extensionsgurt bei 24 kreuzend und dann schräg über den Rücken bis zur Verbindung 20 mit dem ersten Extensionsgurt 14.

An dem ersten Extensionsgurt 14 schließt im Bereich der Verbindung 20 ein Verbindungsabschnitt 26 an, der über den Rücken geführt ist. An den Extensionsgurt 16 schließt im Bereich der Verbindung 20 ein Verbindungsabschnitt 28 an, der über die Brust geführt ist. Die beiden Verbindungsabschnitte 26, 28 sind an ihren Enden mit einem Verschlußstück 18 verbindbar. Sowohl die Extensionsgurte 14, 16 als auch die Verbindungsabschnitte 26, 28 sind elastisch dehnbar. Sie sind im Bereich ihrer Kreuzung bzw. 24 Verbindung 20 miteinander vernäht.

Nicht dargestellt ist, daß die Extensionsgurte 14, 16 im Bereich des schlauchförmigen und/oder des kappenförmigen Abschnittes 10, 12 in Taschen geführt sind.

Dargestellt ist jedoch eine Tasche 22, die im Bereich des Schultereckgelenks auf den kappenförmigen Abschnitt 12 aufgenäht ist. Diese dient dazu, eine Silikon-Pelotte aufzunehmen, um so gezielten Druck auf das Schultereckgelenk aufzuüben.

Die Schultergelenkbandage ermöglicht ein einfaches und wirksames Bandagieren der Schulter. Die außen rotierende Wirkung des ersten Extensionsgurtes 14 wird von dem gegenläufig angeordneten zweiten Extensionsgurt 16 aufgehoben. Die willkürliche Außen- und Innenrotation sowie die Abduktion und Elevation werden durch die Extensionsgurte 14, 16 begünstigt. Der entlang des vorderen Seitenrandes des kappenförmigen Abschnitts 12 verlaufende zweite Extensionsgurt stützt das subakrominale Nebengelenk.

## Patentansprüche

1. Schultergelenkbandage mit einem den Oberarm umgreifenden schlauchförmigen Abschnitt (10) aus einem elastischen Material und einem sich an den schlauchförmigen Abschnitt (10) anschließenden, die Schulter abdeckenden kappenförmigen Abschnitt (12) aus einem elastischen Material, ferner einem im äußeren Arm bereich des dem kappenförmigen Abschnitt (12) entfernten Randes (13) des schlauchförmigen Abschnittes (10) an diesen angesetzten, nach vorne wendelförmig um den schlauchförmigen Abschnitt (10) herum unter der Achselhöle der zu bandasierenden Schulter hindurch und an dem hinteren Seitenrand des kappenförmigen Abschnittes (12) entlang und sodann über diezubandasierende Schulter hinwes nach vorne über die Brust verlaufenden ersten Extensionsgurt (14) und mit einem im äußeren Arm bereich des dem kappenförmigen Abschnitt (12) entfernten Randes (13) des schlauchförmigen Abschnittes (10) an diesen angesetzten, nach hinten gegenläufig zu dem ersten Extensionsgurt (14) wendelförmig um den schlauchförmigen Abschnitt (10) und am vorderen Seitenrand des kappenförmigen Abschnittes (12) entlang und sodann über die zu bandasierende Schulter Hinwes unter Kreuzen (24) des ersten Extensionsgurtes (14) über den Rücken verlaufenden und mit dem ersten Extensionsgurt (14) verbindbaren zweiten Extensionsgurt (16), wobei die Extensionsgurte (14, 16) im Bereich ihrer Kreuzung (24) bzw. ihrer Verbindung (20) miteinander vernäht sind und jeder Extensionsgurt (14, 16) einen an die Verbindung anschließenden Verbindungsabschnitt (26,28) aufweist, wobei ein Verbindungsabschnitt (26) den Rücken und der andere Verbindungsabschnitt (28) die Brust überspannt.

2. Schultergelenkbandage nach Anspruch 1, dadurch gekennzeichnet, daß wenigstens das Material des schlauchförmigen Abschnitts (10) längselastisch ausgebildet ist.

3. Schultergelenkbandage nach Anspruch 1 oder 2, dadurch gekennzeichnet, daß die beiden Verbindungsabschnitte (26, 28) über ein eine Längenverstellung ermöglichendes Verschlußstück (18) miteinander verbunden sind.

4. Schultergelenkbandage nach einem der vorangehenden Ansprüche, dadurch gekennzeichnet, daß der schlauchförmige Abschnitt (10) und/oder der kappenförmige Abschnitt (12) mit die Extensionsgurte (14, 16) aufnehmende Taschen versehen sind.

5. Schultergelenkbandage nach einem der vorangehenden Ansprüche, dadurch gekennzeichnet, daß der kappenförmige Abschnitt (12) im Bereich des Schultereckgelenks mit einer zur Aufnahme einer Pelotte dienenden Tasche (22) versehen ist.

## Claims

1. Bandage for shoulder articulation, comprising a tubular section (10) of a resilient material which embraces the upper arm and a cap-shaped section (12) of a resilient material which is adjacent to the tubular section (10) and covers the shoulder, furthermore a first extension strap (14) which is fitted to the tubular section (10) in the outer arm region of its edge (13) remote from the cap-shaped section (12), is wound helically around the tubular section (10) towards the front below the armpit of the shoulder to be bandaged, extends along the rear side edge of the cap-shaped section (12) and then extends over the shoulder to be bandaged towards the front over the chest, and a second extension strap (16) which is fitted to the tubular section (10) in the outer arm region of its edge (13) remote from the cap-shaped section (12), is wound helically around the tubular section (10) towards the rear in the opposite direction to the first extension strap (14), extends along the front side edge of the cap-shaped section (12) and then extends over the shoulder to be bandaged over the back, thereby intersecting (24) the first extension strap (14), and can be connected to the first extension strap (14), the extension straps (14, 16) being sewn together in the region of their intersection (24) or connection (20) and each extension strap (14, 16) having a connecting section (26, 28) adjacent to the connection, one connecting section (26) crossing the back and the other connecting section (28) crossing the chest.

2. Bandage for shoulder articulation according to claim 1, characterised in that at least the material of the tubular section (10) is longitudinally resilient.

3. Bandage for shoulder articulation according to claim 1 or claim 2, characterised in that the two connecting sections (26, 28) are connected together by a closure member (18) allowing for longitudinal adjustment.

4. Bandage for shoulder articulation according to one of the preceding claims, characterised in that the tubular section (10) and/or the cap-shaped section (12) are provided with pockets receiving the extension straps (14, 16).

5. Bandage for shoulder articulation according to one of the preceding claims, characterised in that the cap-shaped section (12) is provided in the region of the scapuloclavicular articulation with a pocket (22) serving to receive a pad.

## Revendications

1. Bandage pour l'articulation de l'épaule comportant une section (10) de forme tubulaire, constituée d'un matériau élastique, entourant le bras, et une section (12) en forme de coiffe constituée d'un matériau élastique, couvrant l'épaule, se raccordant à section (10) de forme tubulaire, ledit bandage comportant en outre une première sangle d'extension (14) accolée contre la section (10), s'étendant dans la zone extérieure de bras du bord (13) de la section (10) de forme tubulaire, éloigné de la section (12) en forme de coiffe, de manière hélicoïdale, vers l'avant autour de la section (10) de forme tubulaire en passant sous l'aisselle de l'épaule à bander et le long du bord latéral arrière de la section (10) en forme de coiffe et s'étendant ainsi sur la poitrine en revenant vers l'avant par dessus l'épaule à bander, et une deuxième sangle d'extension (16) accolée contre la section (10) et s'étendant dans la zone extérieure de bras du bord (13) de la section (10) de forme tubulaire, éloigné de la section (12) en forme de coiffe, vers l'arrière dans le sens opposé par rapport à la première sangle d'extension (14), de manière hélicoïdale, autour de la section (10) de forme tubulaire et le long du bord latéral avant de la section (12) en forme de coiffe et s'étendant ainsi sur le dos en passant par dessus l'épaule à bander et en croisant (24) la première sangle d'extension (14) et pouvant être reliée à la première sangle d'extension (14), les sangles d'extension (14, 16) étant cousues l'une à l'autre dans leur zone de croisement (24) ou de liaison (20) et chaque sangle d'extension (14, 16) présentant une section de liaison (26, 28) se raccordant à la liaison, une section de liaison (26) passant sur le dos et une autre section de liaison (28) passant sur la poitrine.

2. Bandage pour l'articulation de l'épaule selon la revendication 1, caractérisé en ce qu'au moins le matériau de la section (10) de forme tubulaire est conçu pour être élastique dans le sens longitudinal.

3. Bandage pour l'articulation de l'épaule selon l'une quelconque des revendications 1 ou 2 caractérisé en ce que les deux sections de liaison (26, 28) sont reliées l'une à l'autre par une pièce de raccord (18) permettant un réglage de la longueur.

4. Bandage pour l'articulation de l'épaule selon l'une quelconque des revendications précédentes, caractérisé en ce que la section (10) de forme tubulaire et/ou la section (12) en forme de coiffe sont pourvues de poches pour loger les sangles d'extension (14, 16).

5. Bandage pour l'articulation de l'épaule selon l'une quelconque des revendications précédentes, caractérisé en ce que la section (12) en forme de coiffe est pourvue, dans la zone saillante de l'articulation de l'épaule, d'une poche (22) servant à loger un tampon.
